(19) Europäisches Patentamt
European Patent Office
Office européen des brevets

(11) Veröffentlichungsnummer: **0 002 482**
**B1**

(12) # EUROPÄISCHE PATENTSCHRIFT

(45) Veröffentlichungstag der Patentschrift: **07.01.81**

(51) Int. Cl.³: **C 07 D 417/12**, C 07 C 91/12, A 61 K 31/54

(21) Anmeldenummer: **78101543.3**

(22) Anmeldetag: **04.12.78**

(54) **4-Hydroxy-2H-1,2-benzothiazin-3-carboxamid-1,1-dioxide, Verfahren zu ihrer Herstellung und diese Verbindungen enthaltende Arzneimittel.**

(30) Priorität: **16.12.77 DE 2756113**

(43) Veröffentlichungstag der Anmeldung: **27.06.79 Patentblatt 79/13**

(45) Bekanntmachung des Hinweises auf die Patenterteilung: **07.01.81 Patentblatt 81/1**

(84) Benannte Vertragsstaaten: **BE CH DE FR GB IT LU NL SE**

(56) Entgegenhaltungen: **LU-A-69 905**

(73) Patentinhaber: **Dr. Karl Thomae GmbH, Postfach 720, D-7950 Biberach (Riss) (DE)**

(72) Erfinder: **Trummlitz, Günter, Dr. Dipl.-Chem., Mozartstrasse 4, D-7950 Biberach 1 (DE)**
Erfinder: **Engel, Wolfhard, Dr. Dipl.-Chem., Mozartstrasse 13, D-7950 Biberach 1 (DE)**
Erfinder: **Seeger, Ernst, Dr. Dipl.-Chem., Alpenstrasse 39, D-7950 Biberach 1 (DE)**
Erfinder: **Engelhardt, Günther, Dr., Unterer Bühl 18, D-7950 Biberach 1 (DE)**

**EP 0 002 482 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

# 4-Hydroxy-2H-1,2-benzothiazin-3-carboxamid-1,1-dioxide, Verfahren zu ihrer Herstellung und diese Verbindungen enthaltende Arzneimittel

Die Erfindung betrifft neue 4-Hydroxy-2H-1,2-benzothiazin-3-caraboxamid-1,1-dioxide der allgemeinen Formel I,

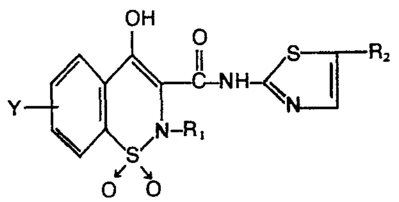

(I)

ihre physiologisch verträglichen Salze mit anorganischen oder organischen Basen, Verfahren zu ihrer Herstellung und diese enthaltende Arzneimittel. Unter den Salzen mit organischen Basen kommt den N-Methyl-D-glucaminsalzen eine besondere Bedeutung zu, da sich diese zur Herstellung injizierbarer Lösungen gut eignen.

In der obigen allgemeinen Formel I bedeuten:
$R_1$ ein Wasserstoffatom oder eine Methyl- oder Äthylgruppe,
$P_2$ eine Methyl-, Äthyl- oder n-Propylgruppe und
Y ein Wasserstoffatom, die Methyl- oder Methoxygruppe oder ein Fluor- oder Chloratom.

Die US-Patentschrift 3591584 bzw. die LU-A-69905 beschreiben 3,4-Dihydro-2H-1,2-benzothiazin-1,1-dioxide, die mit den Verbindungen der allgemeinen Formel I verwandt sind. Es hat sich aber überraschenderweise herausgestellt, dass die Verbindungen der allgemeinen Formel I auch den konstitutionell am nächsten verwandten Verbindungen dieser Publikationen hinsichtlich ihrer antiphlogistischen Wirkung und ihrer Verträglichkeit signifikant überlegen sind.

Die Verbindungen der allgemeinen Formel I lassen sich nach folgenden Verfahren erhalten:

1. Sämtliche Verbindungen der allgemeinen Formel I lassen sich durch Umsetzung von 4-Hydroxy-2H-1,2-benzothiazin-1,1-dioxid-3-carbonsäure-derivaten der allgemeinen Formel II,

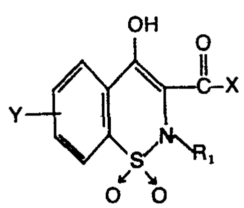

(II)

in der X eine nucleophil austauschbare Gruppe, insbesondere eine Alkoxygruppe mit 1 bis 8 Kohlenstoffatomen, eine Phenylalkoxygruppe mit insgesamt 7 bis 10 Kohlenstoffatomen, die Phenyloxygruppe, ein Halogenatom, die freie Aminogruppe, eine Alkylaminogruppe mit 1 bis 8 Kohlenstoffatomen, eine Cycloalkylaminogruppe mit 3 bis 10 Kohlenstoffatomen, eine Phenylalkylaminogruppe mit insgesamt 7 bis 10 Kohlenstoffatomen oder die Anilinogruppe bedeutet,
und
$R_1$ und Y wie oben definiert sind, mit einem aromatischen Amin der allgemeinen Formel III,

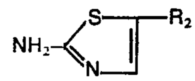

(III)

in der
$R_2$ die oben angegebenen Bedeutungen aufweist, erhalten.

Die Reaktion der Carbonsäureester der allgemeinen Formel II mit den aromatischen Aminen der allgemeinen Formel III erfolgt in geeigneten, indifferenten organischen Lösungsmitteln, beispielsweise in aromatischen Kohlenwasserstoffen wie Benzol, Toluol, Xylol, Chlorbenzol, o-Dichlorbenzol oder Tetrahydronaphthalin, in Dimethylformamid, Dimethylacetamid oder Dimethylsulfoxid oder in Hexamethylphosphorsäuretriamid, in Äthern, wie Dimethoxyäthan, Diäthylenglykoldimethyläther oder Diphenyläther oder auch direkt im überschüssigen Amin. Man arbeitet bei einer Temperatur von 60 bis 200°C, sofern X in der allgemeinen Formel II eine Alkoxygruppe ist, zwischen 20 und 180°C. Vorzugsweise setzt man in Toluol oder Xylol bei Siedetemperatur um und entfernt, sofern in der allgemeinen Formel II X eine Alkoxygruppe, Phenylalkoxygruppe oder Phenyloxygruppe bedeutet, den bei der Reaktion entstehenden Alkohol oder Phenol durch azeotrope Destillation oder durch Erhitzen unter Rückfluss beispielsweise unter Verwendung eines mit Molekularsieb beschickten Soxhlet-Extraktors. Das Produkt kristallisiert direkt aus dem Reaktionsgemisch aus oder wird bei Verwendung eines mit Wasser mischbaren Lösungsmittels durch Zugabe von Wasser ausgefällt. Ist X in der allgemeinen Formel II die Aminogruppe oder eine, wie oben angegeben, substituierte Aminogruppe, so fügt man bei der Umsetzung vorteilhafterweise noch eine katalytische Menge von p-Toluolsulfonsäure zu und setzt das aromatische Amin im Überschuss ein. Auch hier kristallisiert das Produkt oft direkt aus dem Reaktionsgemisch aus, man erhält es aber in jedem Fall durch Abdampfen des Lösungsmittels; es kann aber auch bei Verwendung eines mit Wasser mischbaren Lösungsmittels durch Zugabe von Wasser ausgefällt werden.

2. Verbindungen der allgemeinen Formel I, in der $R_1$ eine Methyl- oder Äthylgruppe bedeutet und in der $R_2$ und Y die eingangs definierte Bedeutung haben, lassen sich auch durch Umsetzung eines 4-Hydroxy-2H-1,2-benzothiazin-3-carboxamid-1,1-dioxids der allgemeinen Formel IV,

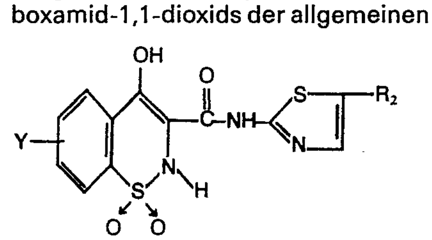

(IV)

in der
$R_2$ und Y wie oben definiert sind, mit einem Alkylhalogenid der allgemeinen Formel V,

$R_{11}$ - Hal (V)

in der
Hal ein Halogenatom und
$R_{11}$ eine Methyl- oder Äthylgruppe bedeutet, in Gegenwart von Basen, erhalten.

Als Basen können Alkali- oder Erdalkalihydroxide, beispielsweise Natrium-, Kalium- oder Bariumhydroxid oder Alkali- oder Erdalkalicarbonate, wie Natrium- oder Kaliumcarbonat sowie Alkali- oder Erdalkalimetallalkoholate, beispielsweise Natriummethylat, Kaliumäthylat, Kalium-tert.butylat oder tertiäre Amine, beispielsweise Triäthylamin eingesetzt werden, sofern man in wässrigem Medium, in alkoholischem Medium, etwa in Methanol, Äthanol, n-Propanol, iso-Propanol oder in Mischungen aus den genannten Lösungsmitteln arbeitet.

Das Alkylhalogenid, vorzugsweise eine Alkylbromid oder -jodid, wird zweckmässig in alkoholischer Lösung direkt zu den übrigen Komponenten in das Reaktionsgemisch gegeben, wobei im Falle des Methylbromids in einer geschlossenen Apparatur gearbeitet wird. Als weitere Lösungsmittel kommen in Frage: Dimethylformamid, Dimethylacetamid, Dimethylsulfoxid, Hexamethylphosphorsäuretriamid.

Sofern man Alkali- oder Erdalkalicarbonate als Basen verwendet, kommen als Lösungsmittel auch aliphatische Ketone, wie Aceton, in Betracht.

Wird die Reaktion in aprotischen organischen Lösungsmitteln, wie z.B. in Benzol oder einem anderen aromatischen Kohlenwasserstoff, in Tetrahydrofuran oder einem anderen offenkettigen oder cyclischen Äther durchgeführt, so kann man als Basen auch Alkalimetallhydride oder Erdalkalimethallhydride, z.B. Natriumhydrid, verwenden. Dabei erfolgt die Zugabe des Alkylhalogenids jedoch erst, wenn sich das Alkalimetallhydrid bzw. Erdalkalimetallhydrid vollständig mit der Ausgangsverbindung der allgemeinen Formel IV umgesetzt hat. Die Reaktionstemperatur beträgt 0 bis 80°C.

In manchen Fällen empfiehlt es sich, vor der Durchführung der beiden, vorstehend genannten Verfahren die 4-Hydroxygruppe in den Verbindungen der allgemeinen Formeln II oder IV durch eine Schutzgruppe zu schützen, wobei nach der Beendigung der Umsetzung diese Schutzgruppe wieder abgespalten wird. So ist beispielsweise eine Verätherung der 4-Hydroxygruppen vorteilhaft; man führt diese Hydroxygruppen in an sich bekannter Weise in die entsprechenden Alkoxy- oder Phenylalkoxygruppen über, beispielsweise in Alkoxygruppen mit 1 bis 8 Kohlenstoffatomen oder Phenylalkoxygruppen mit insgesamt 7 bis 10 Kohlenstoffatomen und spaltet nach der Umsetzung diese Schutzgruppen, beispielsweise durch Erhitzen in Mineralsäuren, wie Bromwasserstoffsäure, auf Temperaturen bis zu 100°C, oder durch Zugabe von Bortrihalogeniden, wie Bortribromid oder Bortrichlorid, in inerten Lösungsmitteln, wie chlorierten Kohlenwasserstoffen, bei Temperaturen zwischen −80°C und +80°C wieder ab.

Die Verbindungen der allgemeinen Formel I können gewünschtenfalls nach an sich bekannten Methoden in ihre physiologisch verträglichen Salze mit anorganischen oder organischen Basen übergeführt werden. Als Basen kommen beispielsweise in Betracht:
Alkalialkoholate, Alkalihydroxide, Erdalkalihydroxide, Trialkylammoniumhydroxide, Alkylamine, vorzugsweise Aminopolyalkohole, insbesondere aber das N-Methyl-D-glucamin.

Die als Ausgangsverbindungen dienenden Ester der allgemeinen Formel II, in der X einen Alkoxy-, Phenylalkoxy- oder Phenoxyrest bedeutet, sind allgemein bekannt und können zum Beispiel nach der deutschen Offenlegungsschrift 1943265 (vgl. auch US-Patentschrift 3591584) hergestellt werden; so geht man beispielsweise von den bekannten 3-Oxo-1,2-benzisothiazol-2(3H)-essigsäureester-1,1-dioxiden (Chem. Berichte 30, 1267 [1897]) aus und gibt zu diesen ein Alkalimetallalkoholat, beispielsweise Natriumäthanolat, in einem organischen polaren Lösungsmittel wie Dimethylsulfoxid oder Dimethylformamid. Es setzt dabei eine Umlagerungsreaktion ein, wobei nach dem Ansäuren der entsprechende Ester der Formel II erhalten wird, in der $R_1$ Wasserstoff bedeutet. Will man in 2-Stellung dieses Esters die anderen für $R_1$ oben erwähnten Gruppen einführen, so geschieht dies am vorteilhaftesten mittels eines Alkylhalogenids, vorzugsweise mit Hilfe eines Alkyljodids; die Alkylierung erfolgt in Gegenwart einer Base.

Die Ausgangsverbindungen der allgemeinen Formel II, in der X eine Aminogruppe oder substituierte Aminogruppe bedeutet, sind literaturbekannt; sie lassen sich z.B. gemäss den Angaben der deutschen Offenlegungsschrift 1943265 (vgl. auch U.S.-Patentschrift Nr. 3591584) aus den 4-Hydroxy-2H-1,2-benzothiazin-3-carbonsäureester-1,1-dioxiden der allgemeinen Formel II durch Umsetzung mit Aminen der allgemeinen Formel $NH_2$–$R_4$, in der $R_4$ ein Wasserstoffatom, eine Alkylgruppe mit 1 bis 8 Kohlenstoffatomen, eine Cycloalkylgruppe mit 3 bis 10 Kohlenstoffatomen, eine Phenylalkylgruppe mit insgesamt 7 bis 10 Kohlenstoffatomen oder die Phenylgruppe bedeutet, in einem indifferenten Lösungsmittel, wie Dimethylsulfoxid oder tert.Butanol, bei Temperaturen zwischen 20 und 200°C herstellen.

Die Ausgangsverbindungen der allgemeinen Formel II, in der X Halogen bedeutet, erhält man beispielsweise durch Umsetzung eines entsprechenden 4-Hydroxy- oder 4-Alkoxy-2H-1,2-benzothiazin-3-carbonsäure-1,1-dioxids mit einem Thionylhalogenid in einem Lösungsmittel, wie Benzol und Dimethylformamid, bei Temperaturen bis zum Rückfluss des Reaktionsgemisches.

Die Verbindungen der allgemeinen Formel III sind ebenfalls literaturbekannt (vgl. H. Erlenmeyer, Z. Herzfeld und B. Prijs, Helv. chim. Acta 38, 1291 [1955] oder K.D. Kalkarni und M.V. Shirsat, J. Sci. and Ind. Research (India), 18B, 411 [1959]; C.A. 54, 14230d [1960]).

Die Ausgangsverbindungen der allgemeinen Formel IV stellt man z.B. aus 4-Hydroxy-2H-1,2-benzothiazin-3-carbonsäureester-1,1-dioxiden der allgemeinen Formel II, in der $R_1$ Wasserstoff bedeutet, durch Umsetzung mit aromatischen Aminen der allgemeinen Formel III in geeigneten indifferenten organischen Lösungsmitteln bei Temperaturen zwischen 20 bis 180°C her.

Wie eingangs erwähnt, besitzen die 4-Hydroxy-2H-1,2-benzothiazin-3-carboxamid-1,1-dioxide der allgemeinen Formel I und ihre physiologisch verträglichen Salze mit anorganischen oder organischen Basen wertvolle pharmakologische Eigenschaften. Diese Verbindungen wirken stark entzündungshemmend, mildern den Entzündungsschmerz, sind für die Behandlung rheumatischer Erkrankungen besonders geeignet und zeigen antithrombotische Wirkungen. Die Verbindungen der allgemeinen Formel I zeigen, im Gegensatz zu den nächstverwandten Verbindungen der US-Patentschrift 3591584 (vgl. auch LU-A-69905), bei einer Dosierung, bei welcher sich ihr entzündungshemmender Effekt schon voll auswirkt, noch keine schädigende Wirkung auf die Magenschleimhaut.

Es wurde beispielsweise die Substanz
4-Hydroxy-2-methyl-N-(5-methyl-2-thiazolyl)-2H-1,2-benzothiazin-3-carboxamid-1,1-dioxid des Beispiels 1 = A,
vergleichend mit dem durch das US Patent 3591584 (vgl. auch LU-A-69905) bekannten
4-Hydroxy-2-methyl-N-(4-methyl-2-thiazolyl)-2H-1,2-benzothiazin-3-carboxamid-1,1-dioxid = B,
4-Hydroxy-2-methyl-N-(2-thiazolyl)-2H-1,2-benzothiazin-3-carboxamid-1,1-dioxid (Sudoxicam) = C
und
N-(4,5-Dimethyl-2-thiazolyl)-4-hydroxy-2-methyl-2H-1,2-benzothiazin-3-carboxamid-1,1-dioxid = D
an der Ratte nach oraler Gabe auf ihre antiphlogistische Wirkung gegenüber der Adjuvansarthritis, auf ihre Wirkung gegen den Entzündungsschmerz in der Versuchsanordnung nach Randall-Selitto sowie auf ihre ulcerogene Wirkung am Magen der Ratte untersucht. Darüberhinaus wurde ihre akute Toxizität an der Maus nach oraler Gabe bestimmt.

Bestimmung der Wirkung gegenüber der Adjuvansarthritis der Ratte:

Männliche Chbb: Thom-Ratten bzw. Lewis-Ratten mit einem mittleren Gewicht zu Versuchsbeginn von 210 g erhielten 0,1 ml einer 1%igen Suspension von M. butyricum in zähflüssigem Paraffinöl in die rechte Hinterpfote subplantar injiziert.

Die Prüfsubstanzen wurden beginnend mit der M. butyricum-Injektion einmal täglich als Verreibung in 1%iger Methylzellulose (1 ml/100 g Tier) für die Dauer von 20 Tagen per Schlundsonde beigebracht.

Am 21. Tage nach der Auslösung der Adjuvansarthritis wurden die Volumina der rechten Hinterpfote (an denen sich eine entzündliche Primärrekation ausgebildet hatte) sowie der linken Hinterpfote (an denen es zur spezifischen immunologisch bedingten Entzündungsreaktion gekommen war) der mit Prüfsubstanz behandelten Tiere mit denen der scheinbehandelten Kontrolltiere verglichen.

Nach linearer Regressionsanalyse wurde eine $ED_{50}$ mit den Vertrauensgrenzen nach Fieller (Quart. J. Pharm. Pharmecol. 17, 117 [1944]) als die Dosis berechnet, die zu einer Reduktion der Pfotenschwellung um 50% gegenüber der bei den Kontrolltieren beobachteten geführt hatte.

Bestimmung der ulcerogenen Wirkung am Magen der Ratte:

Männliche Chbb: Thom-Ratten mit einem mittleren Gewicht von 130 g zu Versuchsbeginn, die mit einer Standarddiät (Altromin-R) ad libitum ernährt wurden, erhielten die Prüfsubstanz als Verreibung in 1%iger Methylzellulose (1 ml/100 g Tier) an 3 aufeinanderfolgenden Tagen einmal täglich per Schlundsonde.

4 Stunden nach der letzten Applikation wurden die Tiere getötet. Die Mägen wurden präpariert und die Schleimhaut nach Abspülen makroskopisch beurteilt.

Aus dem Prozentsatz der Tiere, die mindestens einen Ulcus bzw. eine hämorrhagische Erosion der Magenschleimhaut aufzuweisen hatten, wurden nach Litchfield u. Wilcoxon (J. Pharmacol. exp. Therap. 96, 99 [1949]) eine $ED_{50}$ berechnet.

Bestimmung der akuten Toxizität

Die Bestimmung der akuten Toxizität erfolgte an männlichen und weiblichen Chbb: NMRI (SPF)-Mäusen (in jeder Dosisgruppe zu gleichen Teilen) in einem mittleren Gewicht von 20 g. Die Tiere erhielten die Prüfsubstanz als Verreibung in 1%iger Methylzellulose (0,2 ml/10 g Tier) per Schlundsonde.

Aus dem Prozentsatz der Tiere, die nach den verschiedenen Dosen innerhalb von 14 Tagen verstarben, wurde nach Litchfield u. Wilcoxon (s.o.) eine $LD_{50}$ berechnet.

Ergebnisse:

Die Ergebnisse dieser Prüfungen sind in den Tabellen 1–3 zusammengestellt.

Die Verbindung A erweist sich gegenüber der entzündlichen Primärreaktion der Ratte am Orte der Injektion des Adjuvans als etwa 3 mal wirksamer als die Verbindung C. Gegenüber der spezifischen, immunologisch bedingten Entzündungsreaktion an der kontralateralen Pfote (spezifische Sekundärreaktion) ist A etwa 5 mal wirksamer als C. Dennoch ist die Magenverträglichkeit von A wesentlich besser als die von C. C ist trotz der schwächeren antiphlogistischen Wirksamkeit am Magen der Ratte 2 mal stärker ulcerogen als A. Die therapeutische Breite von A ist nahezu 7 mal grösser als die von C (siehe Tabelle 4).

Die Verbindung D enspricht in ihrer entzündungswidrigen Wirksamkeit weitgehend der Substanz C (Sudoxicam) und erreicht damit bei

dem Vergleich auf der Basis der $ED_{50}$-Werte etwa ein Viertel der antiphlogistischen Wirksamkeit der Verbindung A.

Verbindung B erreicht nicht voll die antiphlogistische Wirksamkeit der Verbindung A. Der entscheidende Nachteil von B ist aber die starke Ulcerogenität am Magen (mehr als 6 mal stärker als bei A). Da die ulcerogene Wirkung von B relativ stärker ausgeprägt ist als die antiphlogistische Wirkung, lässt sich B nicht als Antiphlogisticum therapeutisch verwenden.

Der therapeutische Index von B ist noch geringer als der von C (siehe Tabelle 4). Die therapeutische Breite von A ist 10mal grösser als die von B.

In der akuten Toxizität besteht zwischen den 4 Substanzen keine signifikanten Differenzen (siehe Tabelle 3). Dies bedeutet, dass der Abstand zwischen den antiphlogistisch wirksamen Dosen und den toxischen Dosen bei der Substanz A deutlich grösser ist als bei den drei anderen Verbindungen (siehe Tabelle 5). Dieser Befund ist jedoch von geringerer Bedeutung. Bei der therapeutischen Anwendung von nichtsteroidischen Antiphlogistica wirkt sich nicht die akute Toxizität dosislimitierend aus. Bei dieser Gruppe von Pharmaka ist es vielmehr die ulcerogene Wirkung am Magen-Darm-Kanal, die bei der Anwendung über längere Zeit die Tagesdosis begrenzt.

Tabelle 1

Vergleich der Wirksamkeit gegen die Adjuvansarthritis der Ratte nach täglicher oraler Gabe für die Dauer von 21 Tagen auf der Basis der $ED_{50}$

| Substanz | Wirkung gegen die Primärreaktion $ED_{50}$ mg/kg +) | Wirkung gegen die Sekundärreaktion $ED_{50}$ mg/kg +) |
|---|---|---|
| A | 0,28 (0,14–0,61) | 0,12 (0,09–0,14) |
| Vergleichssubstanzen | | |
| B | 0,37 (0,30–0,48) | 0,21 (0,15–0,28) |
| C | 0,77 (0,60–0,88) | 0,60 (0,45–0,83) |
| D | 1,13 (1,00–1,28) | 0,46 (0,40–0,53) |

+) als Tagesdosis

Tabelle 2

Ulcerogene Wirkung am Magen der Ratte nach täglicher oraler Gabe für die Dauer von 3 Tagen

| Substanz | $ED_{50}$ mg/kg*) |
|---|---|
| A | 2,31 (1,47–3,41) |
| Vergleichssubstanzen | |
| B | 0,36 (0,24–0,54) |
| C | 0,95 (0,53–1,69) |
| D | 1,32 (0,87–2,01) |

*) als Tagesdosis

Tabelle 3

Akute Toxizität an der Maus nach oraler Gabe

| Substanz | $LD_{50}$ mg/kg |
|---|---|
| A | 470 (394–562) |
| Vergleichssubstanzen | |
| B | 488 (287–830) |
| C | 466 (398–545) |
| D | <500*) |

*) nach Verabreichung dieser Dosis verstarben innerhalb von 14 Tagen 6 von 10 Tieren.

Tabelle 4

Vergleich der therapeutischen Breite auf Grund der ulcerogenen Wirkung

| Substanz | I $ED_{50}$ Ulcus mg/kg | II $ED_{50}$ Adjuvans-arthr. Prim.-Reaktion mg/kg | Therapeutischer Index I/II |
|---|---|---|---|
| A | 2,31 | 0,28 | 8,25 |
| Vergleichssubstanzen | | | |
| B | 0,31 | 0,37 | 0,84 |
| C | 0,95 | 0,77 | 1,23 |
| D | 1,32 | 1,13 | 1,17 |

Tabelle 5

Vergleich der therapeutischen Breite auf Grund der $LD_{50}$:

| Substanz | I $LD_{50}$ mg/kg | II $ED_{50}$ Adjuvans-arthr. Prim.-Reaktion mg/kg | Therapeutischer Index I/II |
|---|---|---|---|
| A | 470 | 0,28 | 1679 |
| Vergleichssubstanzen | | | |
| B | 488 | 0,37 | 1319 |
| C | 466 | 0,77 | 605 |
| D | <500 | 1,13 | <460 |

Bei einer vergleichenden Betrachtung der Spalten I und II der Tabelle 4 ergibt sich eindeutig, dass bei den Substanzen B, C und D zur Erzielung entzündungswidriger Effekte Tagesdosen benötigt werden, die bereits eine Schädigung der Magenschleimhaut bewirken.

Die Substanzen A und C wurden des weiteren auf ihre Wirkung gegenüber dem Entzündungsschmerz untersucht:

Die Prüfung der Wirkung gegenüber dem Entzündungsschmerz erfolgte in der Versuchsanordnung nach Randall und Selitto (Arch. int. Pharmacodyn. 111, 409 [1957] an männlichen 100–130 g schweren Chbb: Thom-Ratten. Die Prüfsubstanzen wurden 90 min nach Auslösung des Hefeödems per Schlundsonde beigebracht. Weitere 90 min danach wurde bei mit Prüfsubstanz behandelten Tieren und den nie mit dem Vehikel Methylzellulose behandelten Kontrolltieren die Schmerzschwelle bestimmt und nach linearer Regressionsanalyse eine $ED_{50}$ mit den Vertrauensgrenzen nach Fieller als die Dosis berechnet, die eine Anhebung der Schmerzschwelle um 50% bewirkte.

Die bei diesen Prüfungen erzielten Ergebnisse sind in der nachfolgenden Tabelle 6 zusammengestellt.

Die Substanz A zeichnet sich bei der pharmakologischen Prüfung an der Ratte gegenüber der Substanz C durch eine gesteigerte Wirkung gegenüber dem Entzündungsschmerz aus.

Tabelle 6

| Substanz | Randall-Selitto $ED_{35}$ mg/kg |
|---|---|
| A | 5,6 |
| Vergleichssubstanz C | 9,2 |

Die nachfolgenden Beispiele sollen die Erfindung näher erläutern:

Beispiel 1
4-Hydroxy-2-methyl-N-(5-methyl-2-thiazolyl)-2H-1,2-benzothiazin-3-carboxamid-1,1-dioxid

26,9 g (0,1 Mol) 4-Hydroxy-2-methyl-2H-1,2-benzothiazin-3-carbonsäuremethylester-1,1-dioxid und 12,5 g (0,11 Mol) 2-Amino-5-methyl-thiazol wurden in 4 l Xylol 24 Stunden am Rückfluss in einer Stickstoffatmosphäre erhitzt. Das dabei entstehende Methanol wurde mit 4-Å-Molekularsieb, das sich in einem Soxhlet-Aufsatz befand, entfernt. Die heisse Reaktionslösung wurde filtriert. Aus dem Filtrat kristallisierte beim Abkühlen und Stehen über Nacht das Rohprodukt (32,0 g, 91% der Theorie) aus. Nach Umkristallisation aus Äthylenchlorid wurden 26,0 g (74% der Theorie) 4-Hydroxy-2-methyl-N-(5-methyl-2-thiazolyl)-2H-1,2-benzothiazin-3-carboxamid-1,1-dioxid erhalten; F.: 254°C (Zersetzung);

1H-NMR (/$\bar{D}_6$/-DMSO): δ=8,2–7,8 (m, 4, 5-H bis 8-H);
7,36 (d, 1, J=0,75 Hz, 4′-H); 2,90 (s, 3, $N-CH_3$);
2,36 (d, 3, J=0,75 Hz, 5′-$CH_3$) und 2 austauschbare Protonen.

$C_{14}H_{13}N_3O_4S_2$ (351,40)

| | | | | |
|---|---|---|---|---|
| Ber.: | C47,85 | H3,73 | N11,96 | S18,21 |
| Gef.: | 47,65 | 3,72 | 11,72 | 18,40 |

Beispiel 2
Natriumsalz des 4-Hydroxy-2-methyl-N-(5-methyl-2-thiazolyl)-2H-1,2-benzothiazin-3-carboxamid-1,1-dioxids

Zu einer Lösung von 1,1 g (20 mMol) Natriummethylat in 200 ml Methanol wurden 7,0 g (20 mMol) 4-Hydroxy-2-methyl-N-(5-methyl-2-thiazolyl)-2H-1,2-benzothiazin-3-carboxamid-1,1-dioxid gegeben. Es wurde erwärmt und die entstandene gelbe Lösung filtriert und im Vakuum zur Trockene eingeengt. Der Rückstand wurde mit Aceton und Äther versetzt, abfiltriert und ergab 7,25 g (97,5% der Theorie) 4-Hydroxy-2-methyl-N-(5-methyl-2-thiazolyl)-2H-1,2-benzothiazin-3-carboxamid-1,1-dioxid-natriumsalz; F.: 214°C (Zersetzung).

Beispiel 3
N-Methyl-D-glucaminsalz des 4-Hydroxy-2-methyl-N-(5-methyl-2-thiazolyl)-2H-1,2-benzothiazin-3-carboxamid-1,1-dioxids

6,0 g (17,1 mMol) 4-Hydroxy-2-methyl-N-(5-methyl-2-thiazolyl)-2H-1,2-benzothiazin-3-carboxamid-1,1-dioxid und 3,33 g (17,1 mMol) N-Methyl-D-glucamin wurden in 1 l destilliertem Wasser gelöst. Nach dem Erwärmen auf 60°C wurde die Lösung filtriert. Das Filtrat wurde auf 60 ml im Vakuum eingeengt. Das auskristallisierte 4-Hydroxy-2-methyl-N-(5-methyl-2-thiazolyl)-2H-1,2-benzothiazin-3-carboxamid-1,1-dioxid-N-methyl-D-glucaminsalz wurde abfiltriert und im Vakuum bei 80°C über Phosphorpentoxid getrocknet: 5,2 g (56% der Theorie);

F.: 110°C.

$C_{21}H_{30}N_4O_9S_2$ (546,63)

| | | | | |
|---|---|---|---|---|
| Ber.: | C46,14 | H5,53 | N10,25 | S11,73 |
| Gef.: | 45,95 | 5,76 | 10,24 | 11,98 |

Beispiel 4
4-Hydroxy-N-(5-methyl-2-thiazolyl)-2H-1,2-benzothiazin-3-carboxamid-1,1-dioxid

Hergestellt aus 4-Hydroxy-2H-1,2-benzothiazin-3-carbonsäuremethylester und 2-Amino-5-methyl-thiazol analog dem Beispiel 1. Das Rohprodukt (65% der Theorie) wurde durch Säulenchromatographie (Merck-Kieselgel 60, Korngrösse: 0,2–0,5 mm) unter Verwendung von Chloroform/Äthanol (97:3) als Elutionsmittel gereinigt und ergab 4-Hydroxy-N-(5-methyl-2-thiazolyl)-2H-1,2-benzothiazin-3-carboxamid-1,1-dioxid in einer Ausbeute von 31% der Theorie; F.: 233°C (Zersetzung) aus Äthylenchlorid.

$C_{13}H_{11}N_3O_4S_2$ (337,38

| | | | | |
|---|---|---|---|---|
| Ber.: | C46,29 | H3,29 | N12,45 | S19,01 |
| Gef.: | 46,20 | 3,34 | 12,52 | 19,12 |

Beispiel 5
2-Äthyl-4-hydroxy-N-(5-methyl-2-thiazolyl)-2H-1,2-benzothiazin-3-carboxamid-1,1-dioxid

Hergestellt aus 2-Äthyl-4-hydroxy-2H-1,2-benzothiazin-3-carbonsäuremethylester-1,1-dioxid und 2-Amino-5-methyl-thiazol analog dem Beispiel in einer Ausbeute von 82% der Theorie; F.: 247°C (Zersetzung) aus Xylol.

$C_{15}H_{15}N_3O_4S_2$ (365,43)

| | | | | |
|---|---|---|---|---|
| Ber.: | C49,30 | H4,14 | N11,50 | S17,55 |
| Gef.: | 49,25 | 4,07 | 11,40 | 17,72 |

Beispiel 6

N-(5-Äthyl-2-thiazolyl)-4-hydroxy-2-methyl-2H-1,2-benzothiazin-3-carboxamid-1,1-dioxid

Hergestellt aus 4-Hydroxy-2-methyl-2H-1,2-benzothiazin-3-carbonsäuremethylester-1,1-dioxid und 5-Äthyl-2-amino-thiazol analog dem Beispiel 1 in einer Ausbeute von 67% der Theorie;

F.: 260°C (Zersetzung) aus Xylol.

$C_{15}H_{15}N_3O_4S_2$ (365,43)

| | | | | |
|---|---|---|---|---|
| Ber.: | C49,30 | H4,14 | N11,50 | S17,55 |
| Gef.: | 49,20 | 4,19 | 11,30 | 17,63 |

Beispiel 7

4-Hydroxy-2-methyl-N-(5-n-propyl-2-thiazolyl)-2H-1,2-benzothiazin-3-carboxamid-1,1-dioxid

Hergestellt aus 4-Hydroxy-2-methyl-2H-1,2-benzothiazin-3-carbonsäuremethylester-1,1-dioxid und 2-Amino-5-n-propyl-thiazol in Toluol analog dem Beispiel 1 in einer Ausbeute von 48% der Theorie;

F.: 210°C (Zersetzung) aus Dioxan/Petroläther.

$C_{16}H_{17}N_3O_4S_2$ (379,46)

| | | | | |
|---|---|---|---|---|
| Ber.: | C50,64 | H4,52 | N11,07 | S16,90 |
| Gef.: | 50,90 | 4,64 | 10,97 | 17,00 |

Beispiel 8

2,6-Dimethyl-4-hydroxy-N-(5-methyl-2-thiazolyl)-2H-1,2-benzothiazin-3-carboxamid-1,1-dioxid

4,0 g (14 mMol) 4-Hydroxy-2,6-dimethyl-2H-1,2-benzothiazin-3-carbonsäuremethylester-1,1-dioxid und 2,0 g (17 mMol) 2-Amino-5-methylthiazol wurden in 200 ml wasserfreiem Xylol 24 Stunden am Rückfluss erhitzt. Nach dem Abkühlen wurde das Kristallisat abfiltriert. Die Umkristallisation aus Äthylenchlorid lieferte 3,6 g (70% der Theorie) 2,6-Dimethyl-4-hydroxy-N-(5-methyl-2-thiazolyl)-2H-1,2-benzothiazin-3-carboxamid-1,1-dioxid;

F.: 257°C (Zersetzung);

1H-NMR ($CDCl_3$ + TFA): $\delta$=7,98 (br s, 1, 5-H), 7,92 (d, 1, J=4Hz, 8-H), 7,7 (br d, 1, J=4Hz, 7-H), 7,47 (d, 1, J=1Hz, 4′-H), 2,96 (s, 3, N-$CH_3$) und 2,6 (br s, 6,6-$CH_3$ und 5′-$CH_3$).

$C_{15}H_{15}N_3O_4S_2$ (365,45)

| | | | | |
|---|---|---|---|---|
| Ber.: | C49,30 | H4,14 | N11,50 | S17,55 |
| Gef.: | 49,40 | 4,24 | 11,45 | 17,35 |

Verwendet man anstelle von Xylol als Lösungsmittel o-Dichlorbenzol, Tetrahydronaphthalin oder Diäthylenglykoldimethyläther, so erhält man dieselbe Verbindung in Ausbeuten von 70, 60 respektive 75%.

Die Ausgangsverbindung wurde auf die folgende Weise dargestellt: 45 g (0,23 Mol) 5-Methyl-benzisothiazol-3(2H)-on-1,1-dioxid wurden in eine Lösung von 9,16 g (0,23 Mol) Natriumhydroxid in 500 ml Wasser eingetragen und zum Sieden erhitzt. Die Lösung wurde filtriert und im Vakuum eingeengt. Der Rückstand wurde mehrmals mit Toluol versetzt, das Toluol jeweils abdestilliert und anschliessend mit 200 ml Dimethylsulfoxid und mit 34,72 g (0,32 Mol) Chloressigsäuremethylester versetzt. Das Reaktionsgemisch wurde 3 Stunden auf 120°C erhitzt und nach dem Abkühlen in eine Lösung von 42 g Natriumacetat in 300 ml Wasser eingerührt. Der ausgefallene Niederschlag wurde abgesaugt, mit Wasser gewaschen, erneut mit 200 ml Wasser behandelt, trocken gesaugt und getrocknet. Man erhielt 48,8 g (79% der Theorie) 5-Methyl-3-oxo-benzisothiazol-2(3H)-essigsäuremethylester-1,1-dioxid;

F.: 115°C.

38 g (0,14 Mol) 5-Methyl-3-oxo-benzisothiazol-2(3H)-essigsäuremethylester-1,1-dioxid und 23,9 g (0,44 Mol) Natriummethylat wurden vermischt und mit 250 ml wasserfreiem Toluol und danach mit 42 ml wasserfreiem tert.Butanol unter kräftigem Rühren versetzt. Anschliessend wurde das gelbe Reaktionsgemisch 1 Stunde auf 65°C erwärmt. Nach dem Abkühlen wurde das Reaktionsgemisch auf Eiswasser gegossen und mit Äther versetzt. Die wässrige Phase wurde noch zweimal ausgeäthert und danach vorsichtig mit konzentrierter wässriger Salzsäure angesäuert. Nach erneutem Ausäthern wurde die Ätherphase mit Wasser gewaschen, getrocknet und eingeengt. Der Rückstand wurde aus Essigester umkristallisiert und lieferte 27,6 g (73% der Theorie) 4-Hydroxy-6-methyl-2H-1,2-benzothiazin-3-carbonsäuremethylester-1,1-dioxid;

F.: 169°C.

25 g (0,092 Mol) 4-Hydroxy-6-methyl-2H-1,2-benzothiazin-3-carbonsäuremethylester-1,1-dioxid und 36,9 g (0,26 Mol) Methyljodid wurden in 185 ml Tetrahydrofuran suspendiert und mit einer Lösung von 5,2 g (0,092 Mol) Kaliumhydroxid in 100 ml Wasser versetzt. Nach 24 Stunden wurden weitere 20 g Methyljodid hinzugefügt und nach weiteren 24 Stunden Rühren wurde das 2,6-Dimethyl-4-hydroxy-2H-1,2-benzothiazin-3-carbonsäuremethylester-1,1-dioxid abfiltriert, gewaschen und getrocknet: 9,9 g (38% der Theorie);

F.: 186°C.

Beispiel 9

2,7-Dimethyl-4-hydroxy-N-(5-methyl-2-thiazolyl)-2H-1,2-benzothiazin-3-carboxamid-1,1-dioxid

2,83 g (0,01 Mol) 2,7-Dimethyl-4-hydroxy-2H-1,2-benzothiazin-3-carbonsäuremethylester-1,1-dioxid und 1,25 g (0,011 Mol)2-Amino-5-methylthiazol wurden analog dem Beispiel 8 in Xylol umgesetzt und ergaben 3,1 g (84% der Theorie) 2,7-Dimethyl-4-hydroxy-N-(5-methyl-2-thiazolyl)-2H-1,2-benzothiazin-3-carboxamid-1,1-dioxid;

F.: 228°C (aus Xylol).

Bei Umsetzung in Toluol entstand dasselbe Produkt in 70%iger Ausbeute.

$C_{15}H_{15}N_3O_4S_2$ (365,45)

| | | | | |
|---|---|---|---|---|
| Ber.: | C49,30 | H4,14 | N11,50 | S17,55 |
| Gef.: | 49,25 | 4,08 | 11,41 | 17,62 |

Die Ausgangsverbindung wurde auf die folgende Weise hergestellt: 6-Methyl-benzisothiazol-3(2H)-on-1,1-dioxid wurde analog dem 5-Methyl-benzisothiazol-3(2H)-on-1,1-dioxid (siehe Beispiel 8) mit Natriumhydroxid und Chloressigsäuremethylester zum 6-Methyl-3-oxo-benzisothiazol-2(3H)-essigsäuremethylester-1,1-dioxid (F.: 139°C aus Methanol) umgesetzt. Die nachfolgende Umlagerung mit Natriummethylat in Toluol/tert. Butanol lieferte das 4-Hydroxy-7-methyl-2H-1,2-benzothiazin-3-carbonsäuremethylester-1,1-dioxid, das mit Methyljodid umgesetzt, das 2,7-Dimethyl-4-hydroxy-2H-1,2-benzothiazin-3-carbonsäuremethylester-1,1-dioxid (F.: 183°C) lieferte.

Beispiel 10

4-Hydroxy-t-methoxy-2-methyl-N-(5-methyl-2-thiazolyl)-2H-1,2-benzothiazin-3-carboxamid-1,1-dioxid

5,2 g (0,017 Mol) 4-Hydroxy-6-methoxy-2-methyl-2H-1,2-benzothiazin-3-carbonsäuremethylester-1,1-dioxid und 2,2 g (0,019 Mol) 2-Amino-5-methyl-thiazol wurden in 200 ml Xylol 24 Stunden unter Rückfluss erhitzt. Nach dem Abkühlen wurden die Kristalle abfiltriert und aus Tetrahydrofuran umkristallisiert: 5,8 g (89% der Theorie) 4-Hydroxy-6-methoxy-2-methyl-N-(5-methyl-2-thiazolyl)-2H-1,2-benzothiazin-3-carboxamid-1,1-dioxid;
F.: 260°C;

1H-NMR($CDCl_3$+TFA): δ=7,95 (d, 1, J=4Hz, 8-H), 7,62 (d, 1, J=1, 5Hz, 5-H), 7,45 (d, 1, J=1Hz, 4'-H), 7,35 (dd, 1, J=4Hz und J'=1, 5Hz, 6-H), 4,00 (s, 3, $OCH_3$), 2,95 (s, 3, N-$CH_3$) und 2,55 (d, 3, J=1Hz, 5'-$CH_3$).

$C_{15}H_{15}N_3O_5S_2$ (381,45)

| | | | | |
|---|---|---|---|---|
| Ber.: | C47,23 | H3,96 | N11,02 | S16,81 |
| Gef.: | 47,50 | 4,10 | 10,87 | 16,58 |

Die Ausgangsverbindung wurde auf die folgende Weise hergestellt: 5-Methoxy-benzisothiazol-3(2H)-on-1,1-dioxid wurde analog dem 5-Methyl-benzisothiazol-3(2H)-on-1,1-dioxid (siehe Beispiel 8) mit Natriumhydroxid und Chloressigsäuremethylester zum 5-Methoxy-3-oxo-benzisothiazol-2(3H)-essigsäuremethylester-1,1-dioxid umgesetzt. Die nachfolgende Umlagerung mit Natriummethylat in Toluol/tert.Butanol lieferte das 4-Hydroxy-6-methoxy-2H-1,2-benzothiazin-3-carbonsäuremethylester-1,1-dioxid (F.: 183°C; aus Essigester/Cyclohexan) und die anschliessende Methylierung mit Methyljodid ergab 4-Hydroxy-6-methoxy-2-methyl-2H-1,2-benzothiazin-3-carbonsäuremethylester-1,1-dioxid.
F.: 164°C.

Beispiel 11

6-Chlor-4-hydroxy-2-methyl-N-(5-methyl-2-thiazolyl)-2H-1,2-benzothiazin-3-carboxamid-1,1-dioxid

5,0 g (16,5 mMol) 6-Chlor-4-hydroxy-2-methyl-2H-1,2-benzothiazin-3-carbonsäuremethylester-1,1-dioxid und 2,1 g (18,5 mMol) 2-Amino-5-methyl-thiazol wurden in 300 ml wasserfreiem Xylol 24 Stunden in einer mit 4-Å-Molekularsieb beschickten Soxhlet-Apparatur unter Rückfluss erhitzt. Nach dem Abkühlen wurde das auskristallisierte Rohprodukt abfiltriert und aus Dioxan umkristallisiert: 4,9 g (77% der Theorie) 6-Chlor-4-hydroxy-2-methyl-N-(5-methyl-2-thiazolyl)-2H-1,2-benzothiazin-3-carboxamid-1,1-dioxid;
F.: 285°C (Zersetzung);
1H-NMR(/$\bar{D}_6$/-OMSO): δ8,05 und 7,9 (m, 3, 5-H, 7-H und 8-H); 7,36 (d, 1, J=1Hz, 4'-H); 2,95 (s, 3, N-CH); 2,35 (d, 3, J=1Hz, 5'-$CH_3$) und 2 austauschbare Protonen.

$C_{14}H_{12}N_3O_4S_2$ (385,86)

| | | | | | |
|---|---|---|---|---|---|
| Ber.: | C43,58 | H3,13 | Cl 9,19 | N 10,89 | S16,62 |
| Gef.: | 43,42 | 3,21 | 9,28 | 10,68 | 16,60 |

Die Herstellung der Ausgangsverbindung erfolgt auf die folgende Weise:

43,6 g (0,18 Mol) 5-Chlor-benzisothiazol-3(2H)-on-1,1-dioxidnatriumsalz (hergestellt aus 5-Chlor-benzisothiazol-3(2H)-on-1,1-dioxid und Natronlauge) und 35 ml (0,21 Mol) Chloressigsäuremethylester wurden in 100 ml Dimethylsulfoxid 3 Stunden auf 120°C erwärmt. Nach dem Abkühlen wurden aus dem Reaktionsgemisch 80 ml Dimethylsulfoxid durch Abdestillieren im Vakuum entfernt. Der verbleibende Rückstand wurde in 700 ml Wasser, das 100 g Natriumacetat enthält, eingerührt. Das ausgefallene 5-Chlor-3-oxo-benzisothiazol-2(3H)-essigsäuremethylester-1,1-dioxid wurde abgesaugt, gewaschen und getrocknet (31,1 g entsprechend 60% der Theorie; F.: 118°C).

24,5 g (84,5 mMol) dieser Verbindung wurden mit 13,5 g (253 mMol) Natriummethylat in 190 ml wasserfreiem Toluol (unter Zusatz von 17 ml trokkenem tert.Butanol) auf 80°C für 45 Minuten erwärmt. Das abgekühlte Reaktionsgemisch wurde in Eiswasser eingerührt und mit Äther extrahiert. Die wässrige Phase wurde mit Salzsäure angesäuert. Der weisse Niederschlag wurde abfiltriert, dreimal mit Wasser gewaschen und getrocknet: 14,6 g (60% der Theorie) 6-Chlor-4-hydroxy-2H-1,2-benzothiazin-3-carbonsäuremethylester-1,1-dioxid;
F.: 221°C (Zersetzung).

14,5 g (50 mMol) 6-Chlor-4-hydroxy-2H-1,2-benzothiazin-3-carbonsäuremethylester-1,1-dioxid wurden mit 21,3 g (150 mMol) Methyljodid und 50 ml 1N Natronlauge in 165 ml Methanol umgesetzt und ergaben 12,35 g (81% der Theorie) 6-Chlor-4-hydroxy-2-methyl-2H-1,2-benzothiazin-3-carbonsäuremethylester-1,1-dioxid;
F.: 201°C.

Beispiel 12

7-Fluor-4-hydroxy-2-methyl-N-(5-methyl-2-thia-

zolyl)-2H-1,2-benzothiazion-3-carboxamid-1,1-dioxid

0,29 g (1 mMol) 7-Fluor-4-hydroxy-2-methyl-2H-1,2-benzothiazin-3-carbonsäuremethylester-1,1-dioxid und 0,125 g (1,1 mMol) 2-Amino-5-methyl-thiazol wurden in 50 ml Xylol 24 Stunden am Rückfluss erhitzt. Das Reaktionsgemisch wurde im Vakuum zur Trockene eingeengt und der Rückstand aus Xylol/Cyclohexan umkristallisiert: 0,21 g (57% der Theorie) 7-Fluor-4-hydroxy-2-methyl-N-(5-methyl-2-thiazolyl)-2H-1,2-benzothiazin-3-carboxamid-1,1-dioxid;
F.: 233°C.

Bei Benützung von Benzol als Lösungsmittel wurde nach 30stündigem Erhitzen dieselbe Ausbeute erhalten.

| $C_{14}H_{12}FN_3O_4S_2$ | (369,40) | | | |
|---|---|---|---|---|
| Ber.: | C45,52 | H3,27 | N11,38 | S17,36 |
| Gef.: | 45,40 | 3,18 | 11,42 | 17,18 |

Die Ausgangsverbindung wurde auf die folgende Weise dargestellt: 6-Fluor-benzisothiazol-3(2H)-on-1,1-dioxid wurde analog dem 5-Chlorbenzisothiazol-3(2H)-on-1,1-dioxid (siehe Beispiel 11) mit Natronlauge und mit Chloressigsäuremethylester zum 6-Fluor-3-oxo-benzisothiazol-2(3H)-essigsäuremethylester-1,1-dioxid (F.: 86°C; aus Isopropanol/Petroläther) umgesetzt. Die anschliessende Umlagerungsreaktion mit Natriummethylat lieferte das 7-Fluor-4-hydroxy-2H-1,2-benzothiazin-3-carbonsäuremethylester-1,1-dioxid (F.: 206°C), das mit Methyljodid umgesetzt das 7-Fluor-4-hydroxy-2-methyl-2H-1,2-benzothiazin-3-carbonsäuremethylester-1,1-dioxid (F.: 191°C aus Äthylenchlorid) ergab.

Beispiel 13

4-Hydroxy-2-methyl-N-(5-methyl-2-thiazolyl)-2H-1,2-benzothiazin-3-carboxamid-1,1-dioxid

Hergestellt aus 4-Hydroxy-2-methyl-2H-1,2-benzothiazin-3-carbonsäureäthylester-1,1-dioxid und 2-Amino-5-methyl-thiazol analog dem Beispiel 1, aber unter Verwendung von o-Dichlorbenzol als Lösungsmittel, in einer Ausbeute von 76% der Theorie,
F.: 254°C (Zersetzung) aus Äthylenchlorid,

| $C_{14}H_{13}N_3O_4S_2$ | (351,40) | | | |
|---|---|---|---|---|
| Ber.: | C47,85 | H3,73 | N11,96 | S18,21 |
| Gef.: | 47,91 | 3,78 | 11,80 | 18,42 |

Beispiel 14

4-Hydroxy-2-methyl-N-(5-methyl-2-thiazolyl)-2H-1,2-benzothiazin-3-carboxamid-1,1-dioxid

1,23 g (4,5 mMol) 4-Hydroxy-2-methyl-2H-1,2-benzothiazin-3-carbonsäurechlorid-1,1-dioxid wurden in 10 ml Dimethylformamid gelöst und portionsweise mit 1,0 g (9 mMol) 2-Amino-5-methylthiazol versetzt. Das Reaktionsgemisch wurde 24 Stunden bei Raumtemperatur gerührt und anschliessend mit 40 ml Wasser versetzt. Es wurde 20 Minuten bei Raumtemperatur gerührt und danach wurde der Niederschlag abfiltriert, gewaschen und getrocknet. Umkristallisation aus Äthylenchlorid ergab 0,4 g (25% der Theorie) 4-Hydroxy-2-methyl-N-(5-methyl-2-thiazolyl)-2H-1,2-benzothiazin-3-carboxamid-1,1-dioxid;
F.: 254°C (Zersetzung).

| $C_{14}H_{13}N_3O_4S_2$ | (351,40) | | | |
|---|---|---|---|---|
| Ber.: | C47,85 | H3,73 | N11,96 | S18,21 |
| Gef.: | 47,75 | 3,88 | 11,69 | 17,98 |

Beispiel 15

4-Hydroxy-2-methyl-N-(5-methyl-2-thiazolyl)-2H-1,2-benzothiazin-3-carboxamid-1,1-dioxid

1,0 g (3 mMol) 4-Hydroxy-2-methyl-N-phenyl-2H-1,2-benzothiazin-3-carboxamid-1,1-dioxid wurden mit 1,15 g (10 mMol) 2-Amino-5-methylthiazol und 0,1 g p-Toluolsulfonsäure in 250 ml Xylol 72 Stunden am Rückfluss erhitzt. Nach dem Abkühlen wurde das Reaktionsgemisch mit 2N Salzsäure und anschliessend mit Wasser gewaschen, getrocknet und im Vakuum eingeengt. Der verbleibende Rückstand wurde säulenchromatographisch (Merck-Kieselgel 60; Korngrösse: 0,2–0,5 mm; Elutionsmittel: Chloroform/Äthanol, 95:5) gereinigt und ergab 0,25 g (24% der Theorie) 4-Hydroxy-2-methyl-N-(5-methyl-2-thiazolyl)-2H-1,2-benzothiazin-3-carboxamid-1,1-dioxid;
F.: 254°C (Zersetzung) aus Äthylenchlorid.

| $C_{14}H_{13}N_3O_4S_2$ | (351,40) | | | |
|---|---|---|---|---|
| Ber.: | C47,85 | H3,73 | N11,96 | S18,21 |
| Gef.: | 47,70 | 3,78 | 11,86 | 18,01 |

Beispiel 16

4-Hydroxy-2-methyl-N-(5-methyl-2-thiazolyl)-2H-1,2-benzothiazin-3-carboxamid-1,1-dioxid

Hergestellt aus 4-Hydroxy-2-methyl-2H-1,2-benzothiazin-3-carboxamid-1,1-dioxid und 2-Amino-5-methyl-thiazol und p-Toluolsulfonsäure analog dem Beispiel 15 in einer Ausbeute von 48% der Theorie;
F.: 254°C (aus Äthylenchlorid)

| $C_{14}H_{13}N_3O_4S_2$ | (351,40) | | | |
|---|---|---|---|---|
| Ber.: | C47,85 | H3,73 | N11,96 | S18,21 |
| Gef.: | 47,80 | 3,79 | 12,00 | 18,05 |

Beispiel 17

2-Äthyl-4-hydroxy-N-(5-methyl-2-thiazolyl)-2H-1,2-benzothiazin-3-carboxamid-1,1-dioxid

Zu einer Lösung von 0,7 g (2 mMol) 4-Hydroxy-N-(5-methyl-2-thiazolyl)-2H-1,2-benzothiazin-3-carboxamid-1,1-dioxid in 30 ml Methanol und 2,0 ml 1N Natronlauge wurden 0,94 g (6 mMol) Äthyljodid gegeben. Das Reaktionsgemisch wurde 24 Stunden bei Raumtemperatur gerührt und danach neutralisiert und im Vakuum eingeengt. Der Rückstand wurde säulenchromatographisch (Merck-Kieselgel 60, Korngrösse 0,2–0,5 mm; Elutionsmittel: Chloroform/Äthanol 95:5) gereinigt und ergab nach Umkristallisation aus Xylol 0,35 g (48% der Theorie) 2-Äthyl-4-hydroxy-N-(5-methyl-2-thiazolyl)-2H-1,-

2-benzothiazin-3-carboxamid-1,1-dioxid;
F.: 247°C (Zers.) aus Xylol.

Der Ersatz der Natronlauge durch Kalilauge, Natriummethylat und Kalium-tert.butylat erbrachte ähnliche Ausbeuten.

| | | | | |
|---|---|---|---|---|
| $C_{15}H_{15}N_3O_4S_2$ | (365,43) | | | |
| Ber.: | C49,30 | H4,14 | N11,50 | S17,55 |
| Gef.: | 49,20 | 4,24 | 11,60 | 17,42 |

Beispiel 18

4-Hydroxy-2-methyl-N-(5-methyl-2-thiazolyl)-2H-1,2-benzothiazin-3-carboxamid-1,1-dioxid

Hergestellt aus 4-Hydroxy-N-(5-methyl-2-thiazolyl)-2H-1,2-benzothiazin-3-carboxamid-1,1-dioxid und Methyljodid analog Beispiel 17 in einer Ausbeute von 40% der Theorie; bei Verwendung von Äthanol als Lösungsmittel von 30%.
F.: 254°C (Zersetzung) aus Äthylenchlorid.

| | | | | |
|---|---|---|---|---|
| $C_{14}H_{13}N_3O_4S_2$ | (351,40) | | | |
| Ber.: | C47,85 | H3,73 | N11,96 | S18,21 |
| Gef.: | 48,00 | 3,69 | 12,02 | 18,01 |

Bei Verwendung von Methylbromid erhielt man nach 6stündigem Erhitzen auf Rückflusstemperatur der methanolischen Lösung dasselbe Produkt.

Die Reaktion wurde auch in n-Propanol, Dimethylformamid, Dimethylacetamid und Hexamethylphosphorsäuretriamid bei Temperaturen von 40 bis 60°C durchgeführt, die erzielten Ausbeuten lagen bei 20% (der Theorie).

Beispiel 19

4-Hydroxy-2-methyl-N-(5-methyl-2-thiazolyl)-2H-1,2-benzothiazin-3-carboxamid-1,1-dioxid

0,2 g (0,55 mMol) 4-Methoxy-2-methyl-N-(5-methyl-2-thiazolyl)-2H-1,2-benzothiazin-3-carboxamid-1,1-dioxid wurden mit 1 ml Eisessig und 0,5 ml 48%iger Bromwasserstoffsäure versetzt. Nach 24 Stunden wurde das Reaktionsgemisch 2 Stunden auf dem Wasserbad erwärmt und anschliessend im Vakuum zur Trockene eingeengt. Der Rückstand wurde in Methylenchlorid aufgenommen und mit Wasser gewaschen. Trocknen und Eindampfen der organischen Phase lieferte 4-Hydroxy-2-methyl-N-(5-methyl-2-thiazolyl)-2H-1,2-benzothiazin-3-carboxamid-1,1-dioxid: 0,1 g (52% der Theorie);
F.: 254°C (Zersetzung; aus Äthylenchlorid).

| | | | | |
|---|---|---|---|---|
| $C_{14}H_{13}N_3O_4S_2$ | (351,40) | | | |
| Ber.: | C47,85 | H3,73 | N11,96 | S18,21 |
| Gef.: | 47,82 | 3,67 | 11,80 | 18,01 |

Die Ausgangsverbindung wurde auf die folgende Weise hergestellt: 26,9 g (0,1 Mol) 4-Hydroxy-2-methyl-2H-1,2-benzothiazin-3-carbonsäuremethylester-1,1-dioxid, 85,1 g (0,616 Mol) Kaliumcarbonat und 71 g (0,5 Mol) Methyljodid wurden in 1000 ml Aceton 16 Stunden am Rückfluss erhitzt. Nach jeweils 4 Stunden wurden dem siedenden Reaktionsgemisch je 14 g (0,1 Mol) Methyljodid zugesetzt. Anschliessend wurde das Reaktionsgemisch 12 Stunden bei Raumtemperatur gerührt. Der entstandene Niederschlag wurde abfiltriert und mit Aceton gewaschen. Die Filtrate wurden im Vakuum eingeengt und ergaben nach Unkristallisation aus Tetrachlorkohlenstoff 23,5 g (83% der Theorie) 4-Methoxy-2-methyl-2H-1,2-benzothiazin-3-carbonsäuremethylester-1,1-dioxid;
F.: 78°C.

7,8 g (28 mMol) 4-Methoxy-2-methyl-2H-1,2-benzothiazin-3-carbonsäuremethylester-1,1-dioxid wurden in 75 ml Äthanol gelöst und mit 42 ml 1N Kalilauge versetzt. Das Reaktionsgemisch wurde 6 Stunden am Rückfluss erhitzt, über Nacht bei Raumtemperatur gerührt und anschliessend im Vakuum eingeengt. Der Rückstand wurde in Wasser aufgenommen und ausgeäthert. Die wässrige Phase wurde unter Kühlung angesäuert und der entstandene Niederschlag abfiltriert und mit Wasser gewaschen: 6,3 g (84% der Theorie) 4-Methoxy-2-methyl-2H-1,2-benzothiazin-3-carbonsäure-1,1-dioxid;
F.: 220°C.

6,2 g (23 mMol) 4-Methoxy-2-methyl-2H-1,2-benzothiazin-3-carbonsäure-1,1-dioxid wurden in 60 ml Benzol suspendiert und mit 8,2 ml (0,11 Mol) Thionylchlorid und mit 0,5 ml wasserfreiem Dimethylformamid versetzt. Das Reaktionsgemisch wurde 6 Stunden am Rückfluss erhitzt, über Nacht bei Raumtemperatur gerührt und anschliessend im Vakuum eingedampft. Der Rückstand wurde in wenig Toluol aufgenommen, erneut eingedampft und ergab 6,9 g (100% der Theorie) 4-Methoxy-2-methyl-2H-1,2-benzothiazin-3-carbonsäurechlorid-1,1-dioxid;
F.: 117°C.

Zu einer Lösung von 1,8 g (16 mMol) 2-Amino-5-methyl-thiazol und 1,6 g (16 mMol) Triäthylamin in 100 ml trockenem Benzol tropfte man innerhalb von 1,5 Stunden bei einer Temperatur von 20° bis 30°C eine Lösung von 4,7 g (16 mMol) 4-Methoxy-2-methyl-2H-1,2-benzothiazin-3-carbonsäurechlorid-1,1-dioxid in 150 ml trockenem Benzol. Anschliessend wurde 2 Stunden bei Raumtemperatur und 1 Stunde bei Rückfluss gerührt. Das Reaktionsgemisch wurde heiss filtriert und das Filtrat wurde mit Petroläther versetzt. Beim Abkühlen kristallisierten 3,1 g 2,5-Dimethyl-5H,6H-thiazolo[2′, 3′-2,3]pyrimido[4,5-c]-1,2-benzothiazin-5-on-7-dioxid (F.: 305°C, Zersetzung; aus Essigester) aus.

Aus der Mutterlauge wurden durch Eindampfen zur Trockene und Umkristallisation aus Essigester 1,8 g (31% der Theorie) 4-Methoxy-2-methyl-N-(5-methyl-2-thiazolyl)-2H-1,2-benzothiazin-3-carboxamid-1,1-dioxid erhalten;
F.: 201°C.

| | | | | |
|---|---|---|---|---|
| $C_{15}H_{15}N_3O_4S_2$ | (365,44) | | | |
| Ber.: | C49,30 | H4,14 | N11,50 | S17,55 |
| Gef.: | 49,45 | 4,07 | 11,43 | 17,70 |

Die neuen Verbindungen der allgemeinen Formel I lassen sich zur pharmazeutischen Anwendung in die üblichen pharmazeutischen Zubereitungsformen einarbeiten. Die Einzeldosis beträgt bei Erwachsenen 2 bis 100 mg, vorzugsweise 5 bis 25 mg, die Tagesdosis 5 bis 200 mg, vorzugsweise 10 bis 50 mg.

Die nachfolgenden Beispiele beschreiben die Herstellung einiger pharmazeutischer Zubereitungsformen:

Beispiel I

Tabletten mit 10 mg 4-Hydroxy-2-methyl-N-(5-methyl-2-thiazolyl)-2H-1,2-benzothiazin-3-carboxamid-1,1-dioxid

Zusammensetzung:

1 Tablette enthält

| | |
|---|---|
| Wirksubstanz | 10,0 mg |
| Maisstärke | 112,0 mg |
| Polyvinylpyrrolidon | 175,0 mg |
| Magnesiumstearat | 3,0 mg |
| | 300,0 mg |

Herstellungsverfahren:

Die Mischung der Wirksubstanz mit Maisstärke wird mit einer 14%igen Lösung des Polyvinylpyrrolidons in Wasser durch ein Sieb mit 1,5 mm Maschenweite granuliert, bei 45°C getrocknet und nochmals durch obiges Sieb gerieben. Das so erhaltene Granulat wird mit Magnesiumstearat gemischt und zu Tabletten verpresst.

| | |
|---|---|
| Tablettengewicht: | 300 mg |
| Stempel: | 10 mm, flach |

Beispiel II

Dragées mit 10 mg 4-Hydroxy-2-methyl-N-(5-methyl-2-thiazolyl)-2H-1,2-benzothiazin-3-carboxamid-1,1-dioxid

Zusammensetzung:

1 Dragéekern enthält:

| | |
|---|---|
| Wirksubstanz | 10,0 mg |
| Maisstärke | 260,0 mg |
| Gelatine | 8,0 mg |
| Talk | 18,0 mg |
| Magnesiumstearat | 4,0 mg |
| | 300,0 mg |

Herstellungsverfahren:

Die Mischung der Wirksubstanz mit Maisstärke wird mit einer 10%igen wässrigen Gelatine-Mischung durch ein Sieb der Maschenweite 1,5 mm granuliert, bei 45°C getrocknet und nochmals durch obiges Sieb gerieben. Das so erhaltene Granulat wird mit Talk und Magesiumstearat gemischt und zu Dragéekernen verpresst.

| | |
|---|---|
| Kerngewicht: | 300,0 mg |
| Stempel: | 10 mm, gewölbt |

Die Dragéekerne werden nach bekannten Verfahren mit einer Hülle überzogen, die im wesentlichen aus Zucker und Talkum besteht. Die fertigen Dragées werdenmit Hilfe von Bienenwachs poliert.

Dragéegewicht: 580 mg.

Beispiel III

Gelatine-Kapseln mit 5 mg 4-Hydroxy-2-methyl-N-(5-methyl-2-thiazolyl)-2H-1,2-benzothiazin-3-carboxamid-1,1-dioxid

Zusammensetzung

1 Gelatine-Kapsel enthält:

| | |
|---|---|
| Wirksubstanz | 5,0 mg |
| Maisstärke | 385,0 mg |
| Aerosil | 6,0 mg |
| Magnesiumstearat | 4,0 mg |
| | 400,0 mg |

Herstellungsverfahren:

Die Substanzen werden intensiv gemischt und in Gelatine-Kapseln Grösse 1 abgefüllt.

Kapselinhalt: 400 mg

Beispiel IV

Suppositorien mit 25 mg 4-Hydroxy-2-methyl-N-(5-methyl-2-thiazolyl)-2H-1,2-benzothiazin-3-carboxamid-1,1-dioxid

Zusammensetzung:

1 Zäpfchen enthält:

| | |
|---|---|
| Wirksubstanz | 25,0 mg |
| Suppositorienmasse (z.B. Witepsol W 45) | 1725,0 mg |
| | 1750,0 mg |

Herstellungsverfahren:

Die feinpulverisierte Wirksubstanz wird mit Hilfe eines Entauchhomogenisators in die geschmolzene und auf 40°C abgekühlte Zäpfchenmasse eingerührt. Die Masse wird bei 38°C in leicht vorgekühlte Formen gegossen.

Zäpfchengewicht: 1,75 g

Beispiel V

Suspension mit 25 mg 4-Hydroxy-2-methyl-N-(5-methyl-2-thiazolyl)-2H-1,2-benzothiazin-3-carboxamid-1,1-dioxid in 5 ml

Zusammensetzung:

| | | |
|---|---|---|
| Wirksubstanz | | 0,5 g |
| Dioctylnatrimsulfosuccinat (DONSS) | | 0,02 g |
| Benzoesäure | | 0,1 g |
| Natriumcyclamat | | 0,2 g |
| Aerosil® | | 1,0 g |
| Polyvinylpyrrolidon | | 0,1 g |
| Glycerin | | 25,0 g |
| Grapefruit-Aroma | | 0,1 g |
| Dest. Wasser | ad | 100,0 ml |

Herstellungsverfahren:

In dem auf 70°C erwärmten Wasser werden nacheinander DONSS, Benzoesäure, Natriumcyclamat und Polyvinylpyrrolidon gelöst. Man gibt Glycerin und Aerosil dazu, kühlt auf Raumtemperatur ab und suspendiert mit Hilfe eines Eintauchhomogenisators die feinpulverisierte Wirksubstanz. Anschliessend wird aromatisiert und mit Wasser auf das angegebene Volumen aufgefüllt.

5 ml Suspension enthalten 25 mg Wirksubstanz.

Beispiel VI

Injizierbare 0,2%ige Lösung des N-Methyl-D-glucaminsalzes von 4-Hydroxy-2-methyl-N-(5-methyl-2-thiazolyl)-2H-1,2-benzothiazin-3-carboxamid-1,1-dioxid in Wasser

Zusammensetzung:

| | | |
|---|---|---|
| Wirksubstanz | | 20 mg |
| Natriumchlorid | | 80 mg |
| dest. pyrogenfreies | | |
| Wasser | ad | 10 ml |

Herstellungsverfahren:

Die Substanz wurde in dest. Wasser gelöst, die Lösung wurde dann mit der zur Isotonie-Einstellung erforderlichen Menge Natriumchlorid versetzt und mit dest. Wasser auf das gewünschte Volumen aufgefüllt.

Filtration über Membranfilter (0,2 μm), Abfüllung unter sterilen Bedingungen in sterilisierte Ampullen.

**Patentansprüche**

1. Neue 4-Hydroxy-2H-1,2-benzothiazin-3-carboxamid-1,1-dioxide der allgemeinen Formel I,

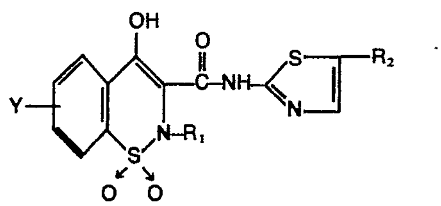

(I)

in der

$R_1$ ein Wasserstoffatom oder eine Methyl- oder Äthylgruppe,

$R_2$ eine Methyl-, Äthyl- oder n-Propylgruppe und

Y ein Wasserstoffatom, die Methyl- oder Methoxygruppe oder ein Fluor- oder Chloratom bedeuten, und deren physiologisch verträgliche Salze mit anorganischen oder organischen Basen.

2. 4-Hydroxy-2-methyl-N-(5-methyl-2-thiazolyl)-2H-1,2-benzothiazin-3-carboxamid-1,1-dioxid und dessen physiologisch verträgliche Salze mit anorganischen oder organischen Basen.

3. Die N-Methyl-D-glucaminsalze der 4-Hydroxy-2H-1,2-benzothiazin-3-carboxamid-1,1-dioxide der allgemeinen Formel I gemäss Anspruch 1.

4. Verfahren zur Herstellung von neuen 4-Hydroxy-2H-1,2-benzothiazin-3-carboxamid-1,1-dioxiden der allgemeinen Formel I

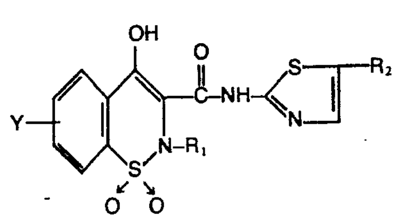

(I)

in der

$R_1$ ein Wasserstoffatom, eine Methyl- oder Äthylgruppe,

$R_2$ eine Methyl-, Äthyl- oder n-Propylgruppe und

Y ein Wasserstoffatom, die Methyl- oder Methoxygruppe oder ein Fluor- oder Chloratom bedeuten, und von deren Salzen mit anorganischen oder organischen Basen, dadurch gekennzeichnet, dass

a) ein 4-Hydroxy-2H-1,2-benzothiazin-1,1-dioxid-3-carbonsäurederivat der allgemeinen Formel II,

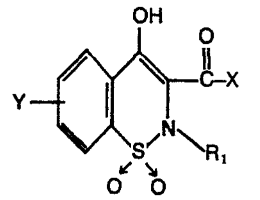

(II)

in der X eine nucleophil austauschbare Gruppe, insbesondere eine Alkoxygruppe mit 1 bis 8 Kohlenstoffatomen, eine Phenylalkoxygruppe mit insgesamt 7 bis 10 Kohlenstoffatomen, die Phenyloxygruppe, ein Halogenatom, die freie Aminogruppe, eine Alkylaminogruppe mit 1 bis 8 Kohlenstoffatomen, eine Cycloalkylaminogruppe mit 3 bis 10 Kohlenstoffatomen, eine Phenylalkylaminogruppe mit insgesamt 7 bis 10 Kohlenstoffatomen oder die Anilinogruppe bedeutet und $R_1$ und Y wie oben definiert sind, mit einem aromatischen Amin der allgemeinen Formel III,

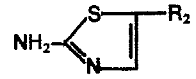

(III)

in der $R_2$ die oben angegebenen Bedeutungen aufweist, in einem indifferenten organischen Lösungsmittel oder in einem Überschuss des Amins der allgemeinen Formel III bei Temperaturen zwischen 20 und 200°C umgesetzt wird oder

b) zur Herstellung von Verbindungen der allgemeinen Formel I, in der $R_1$ die Methyl- oder Äthylgruppe bedeutet und $R_2$ wie eingangs definiert ist, ein 4-Hydroxy-2H-1,2-benzothiazin-3-carboxamid-1,1-dioxid der allgemeinen Formel IV,

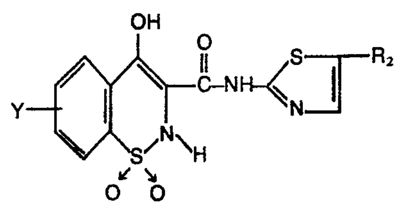

(IV)

in der $R_2$ und Y die oben angegebenen Bedeutungen aufweisen, mit einem Alkylhalogenid der allgemeinen Formel V,

$R_{11}$-Hal (V)

in der Hal ein Halogenatom und $R_{11}$ eine Methyl- oder Äthylgruppe bedeutet, in Gegenwart einer Base bei Temperaturen zwischen 0 und 80°C behandelt wird, und gegebenenfalls vor der Durchführung dieser Verfahren die 4-Hydroxygruppe in Verbindung der allgemeinen Formeln II oder IV durch eine Schutzgruppe geschützt wird, wobei nach der Beendigung der Umsetzung diese Schutzgruppe wieder abgespalten wird, und gewünschtenfalls eine nach den obigen Verfahren

erhaltene Verbindung der allgemeinen Formel I mittels einer anorganischen oder organischen Base anschliessend in ihr Salz übergeführt wird.

5. Verfahren nach Anspruch 4a, dadurch gekennzeichnet, dass, sofern X in der allgemeinen Formel II eine Alkoxygruppe bedeutet, der entstehende entsprechende Alkohol durch azeotrope Destillation entfernt wird.

6. Verfahren nach Anspruch 4b, dadurch gekennzeichnet, dass als Basen Alkali- oder Erdalkalihydroxide, -carbonate oder -alkoholate oder tertiäre Amine in wässerigem, alkoholischem oder wässrig-alkoholischem Medium oder Alkali- oder Erdalkalimetallhydride in aprotischen organischen Lösungsmitteln verwendet werden, wobei bei Verwendung von Alkali- oder Erdalkalicarbonaten auch aliphatische Ketone als Lösungsmittel in Frage kommen.

7. Verfahren nach Anspruch 4a, dadurch gekennzeichnet, dass man, sofern X in der allgemeinen Formel II die Aminogruppe oder eine Alkylamino-, Cycloalkylamino-, Phenylalkylamino- oder Anilinogruppe bedeutet, die Umsetzung in Xylol bei Siedetemperatur durchführt und katalytische Mengen von p-Toluolsulfonsäure zusetzt.

8. Verfahren nach Anspruch 4a und 4b, dadurch gekennzeichnet, dass die freie 4-Hydroxygruppe in einer Verbindung der allgemeinen Formeln II und IV vor der Umsetzung durch Verätherung in eine Alkoxy- oder Phenylalkoxygruppe übergeführt wird und nach der Umsetzung eine derartige Schutzgruppe mittels Mineralsäuren bei Temperaturen zwischen 0 und 100°C oder Bortrihalogeniden bei Temperaturen zwischen −80°C und +80°C wieder abgespalten wird.

9. Arzneimittel, enthaltend eine oder mehrere Verbindungen der allgemeinen Formel I gemäss Anspruch 1 und übliche Träger- und/oder Zusatzstoffe.

**Revendication**

1. Nouveaux 1,1-dioxydes de 4-hydroxy-2H-1,2-benzothiazine-3-carboxamides de formule générale I:

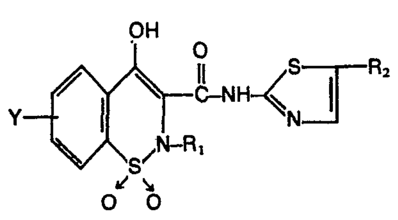

(I)

dans laquelle:
$R_1$ représente un atome d'hydrogène ou un groupe méthyle ou éthyle,
$R_2$ représente un groupe méthyle, éthyle ou n-propyle, et
Y représente un atome d'hydrogène, le groupe méthyle ou méthoxy ou un atome de fluor ou de chlore, et leurs sels physiologiquement supportables avec des bases minérales ou organiques.

2. Le 1,1-dioxyde de 4-hydroxy-2-méthyl-N-(5-méthyl-2-thiazolyl)-2H-1,2-benzothiazine-3-carboxamide et ses sels physiologiquement supportables avec des bases minérales ou organiques.

3. Les sels de N-méthyl-D-glucamine des 1,1-dioxydes de 4-hydroxy-2H-1,2-benzothiazine-3-carboxamides de formule générale I selon la revendication 1.

4. Procédé de préparation des nouveaux 1,1-dioxydes de 4-hydroxy-2H-1,2-benzothiazine-3-carboxamides de formule générale I:

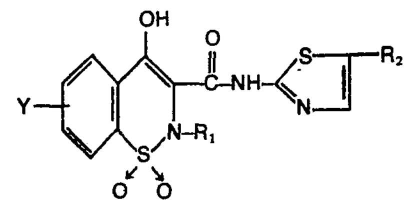

(I)

dans laquelle:
$R_1$ représente un atome d'hydrogène ou un groupe méthyle ou éthyle,
$R_2$ représente un groupe méthyle, éthyle ou n-propyle, et
Y représente un atome d'hydrogène, le groupe méthyle ou méthoxy ou un atome de fluor ou de chlore, ainsi que de leurs sels avec des bases minérales ou organiques, caractérisé en ce que:

a) On fait réagir un dérivé de 1,1-dioxyde d'acide 4-hydroxy-2H-1,2-benzothiazine-3-carboxylique de formule générale II:

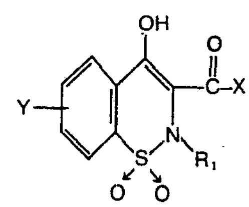

(II)

dans laquelle X représente un groupe échangeable de façon nucléophile, en particulier un groupe alcoxy avec 1 à 8 atomes de carbone, un groupe phénylalcoxy avec en tout 7 à 10 atomes de carbone, le groupe phényloxy, un atome d'halogène, le groupe amino libre, un groupe alcoylamino avec 1 à 8 atomes de carbone, un groupe cycloalcoylamino avec 3 à 10 atomes de carbone, un groupe phénylalcoylamino avec en tout 7 à 10 atomes de carbone ou le groupe anilino et
$R_1$ et Y sont définis comme plus haut, avec une amine aromatique de formule générale II:

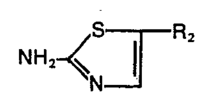

(III)

dans laquelle $R_2$ présente les significations données plus haut, dans un solvant organique indifférent ou dans un excès de l'amine de formule générale III à des températures comprises entre 20 et 200°C ou bien,

b) Pour préparer les composés de formule générale I dans laquelle $R_1$ représente le groupe méthyle ou éthyle et $R_2$ est défini comme au début,

on traite un 1,1-dioxyde de 4-hydroxy-2H-1,2-benzothiazine-3-carboxamide de formule générale IV:

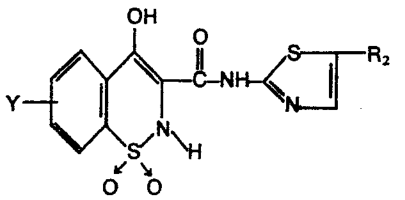

(IV)

dans laquelle $R_2$ et Y présentent les significations données plus haut, avec un halogénure d'alcoyle de formule générale V:

$R_{11}$-Hal (V)

dans laquelle Hal représente un atome d'halogène et $R_{11}$ représente un groupe méthyle ou éthyle en présence d'une base à des températures comprises entre 0 et 80°C, et éventuellement avant la mise en œuvre de ce procédé, on protège le groupe 4-hydroxy dans les composés de formules générales II ou IV au moyen d'un groupe protecteur, ce groupe protecteur étant de nouveau clivé lorsque la réaction est terminée, et si on le désire, on transforme ensuite un composé de formule générale I obtenu selon le procédé ci-dessus en son sel, à l'aide d'une base minérale ou organique.

5. Procédé selon la revendication 4 a), caractérisé en ce que, dans la mesure où X, dans la formule générale II, représente un groupe alcoxy, on élimine l'alcool formé correspondant par distillation azéotropique.

6. Procédé selon la revendication 4 b), caractérisé en ce que l'on utilise comme bases des hydroxydes, des carbonates ou desalcoolates alcalins ou alcalino-terreux ou des amines tertiaires dans un milieu aqueux, alcoolique ou hydroalcoolique ou des hydrures de métaux alcalins ou alcalino-terreux dans des solvants organiques aprotiques, des cétones aliphatiques pouvant également servir de solvants lorsqu'on utilise des carbonates alcalins ou alcalino-terreux.

7. Procédé selon la revendication 4 a), caractérisé en ce que, dans la mesure où X dans la formule générale II représente le groupe amino ou un groupe alcoylamino, cycloalcoylamino, phénylalcoylamino ou anilino, on effectue la réaction dans le xylène à la température d'ébullition et on ajoute des quantités catalytiques d'acide p-toluène-sulfonique.

8. Procédé selon la revendication 4 a) et 4 b), caractérisé en ce que le groupe 4-hydroxy libre dans un composé de formules générales II et IV est transformé avant la réaction en un groupe alcoxy ou phénylalcoxy par éthérification et en ce que, après la réaction, un tel groupe protecteur est de nouveau clivé à l'aide d'acides minéraux à des températures comprises entre 0 et 100°C ou de trihalogénures de bore à des températures comprises entre −80°C et +80°C.

9. Médicament renfermant un ou plusieurs composés de formule générale I selon la revendication 1 et des excipients et/ou additifs habituels.

**Patent Claims**

1. Novel 4-hydroxy-2H-1,2-benzothiazine-3-carboxamide-1,1-dioxides of general formula I

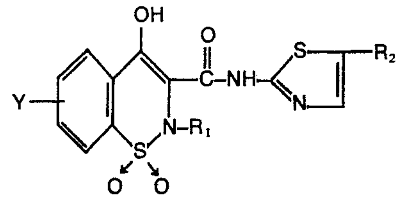

(I)

in which

$R_1$ represents a hydrogen atom or a methyl or ethyl group,

$R_2$ represents a methyl, ethyl or n-propyl group and

Y represents a hydrogen atom, the methyl or methoxy group or a fluorine or chlorine atom, and their physiologically compatible salts with inorganic or organic bases.

2. 4-Hydroxy-2-methyl-N-(5-methyl-2-thiazolyl)-2H-1,2-benzothiazine-3-carboxamide-1,1-dioxide and its physiologically compatible salts with inorganic or organic bases.

3. The N-methyl-D-glucamine salts of 4-hydroxy-2H-1,2-benzothiazine-3-carboxamide-1,1-dioxides of general formula I according to claim 1.

4. Process for the preparation of novel 4-hydroxy-2H-1,2-benzothiazine-3-carboxamide-1,1-dioxides of general formula I

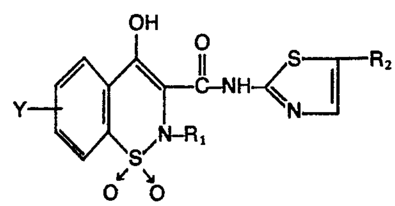

(I)

in which

$R_1$ represents a hydrogen atom, a methyl or ethyl group,

$R_2$ represents a methyl, ethyl or n-propyl group and

Y represents a hydrogen atom, the methyl or methoxy group or a fluorine or chlorine atom, and of their salts with inorganic or organic bases, characterised in that

a) a 4-hydroxy-2H-1,2-benzothiazine-1,1-dioxide-3-carboxylic acid derivative of general formula II

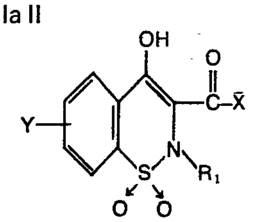

(II)

in which X represents a nucleophilically exchangeable group, especially an alkoxy group with 1 to 8 carbon atoms, a phenylalkoxy group with altogether 7 to 10 carbon atoms, the phenyloxy group, a halogen atom, the free amino group, an alkylamino group with 1 to 8 carbon atoms, a cycloalkylamino group with 3 to 10 carbon atoms, a phenylalkylamino group with altogether 7 to 10 carbon atoms or the anilino group and

$R_1$ and Y are as defined above, is reacted with an aromatic amine of general formula III

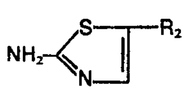

(III)

in which $R_2$ has the meanings mentioned above, in an inert organic solvent or in an excess of the amine of general formula III at temperatures of between 20 and 200°C or

b) for the preparation of compounds of general formula I in which $R_1$ represents the methyl or ethyl group and $R_2$ is as defined above, a 4-hydroxy-2H-1,2-benzothiazine-3-carboxamide-1,1-dioxide of general formula IV

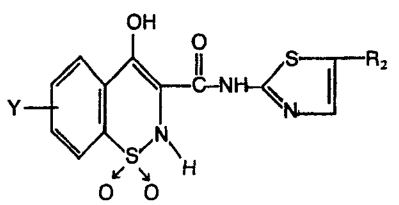

(IV)

in which $R_2$ and Y have the meanings mentioned above, is treated with an alkyl halide of general formula V

$R_{11}$-Hal (V)

in which Hal represents a halogen atom and $R_{11}$ represents a methyl or ethyl group, in the presence of a base at temperatures of between 0 and 80°C, and optionally before these processes are realised the 4-hydroxy group in compounds of general formula II or IV is protected by a protective group, this protective group being split off again after completion of the reaction, and, if desired, a compound of general formula I obtained according to the above processes is subsequently converted into its salt by means of an inorganic or organic base.

5. Process according to claim 4a, characterised in that, if X in general formula II represents an alkoxy group, the corresponding alcohol obtained is removed by azeotropic distillation.

6. Process according to claim 4b, characterised in that the bases used are alkali or alkaline earth hydroxides, carbonates or alcoholates or tertiary amines in aqueous, alcoholic or aqueousalcoholic media or alkali or alkaline-earth metal hydrides in aprotic organic solvents, also aliphatic ketones being considered as solvents in the use of alkali or alkaline earth carbonates.

7. Process according to claim 4a, characterised in that, if X in general formula II represents the amino group or an alkylamino, cycloalkylamino, phenylalkylamino or anilino group, the reaction is effected in xylene at boiling temperature and catalytic quantities of p-toluenesulphonic acid are added.

8. Process according to claim 4a and 4b, characterised in that the free 4-hydroxy group in a compound of general formulae II and IV is converted before the reaction into an alkoxy or phenylalkoxy group by means of etherification and in that after the reaction such a protective group is split off again by means of mineral acids at temperatures of between 0 and 100°C or boron trihalides at temperatures of between −80°C and +80°C.

9. Pharmaceutical agents containing one or more compounds of general formula I according to claim 1 and conventional carriers and/or additives.